# EUROPEAN PATENT APPLICATION

(11) **EP 3 501 392 A1**
(43) Date of publication of application: **26.06.2019**
(21) Application number: 18466005.8
(22) Date of filing: 14.11.2018
(51) Int. Cl.: A61B 5/053, A61B 5/00, G06F 3/0354

(54) **APPARATUS FOR MEASUREMENT OF THE GALVANIC SKIN RESPONSE OF A PERSON WORKING WITH A COMPUTER AND THE METHOD OF MEASURING AND PERFORMING DIAGNOSTICS WITH THIS DEVICE**

(30) Priority: 21.12.2017 CZ 20170826
(71) Applicant: Slamka Consulting, s.r.o., 702 00 Ostrava, Moravska Ostrava (CZ)
(72) Inventor: MAL?ÍK, Martin, 721 00 Ostrava, Svinov (CZ)
(74) Representative: Soukup, Petr

(57) **Abstract**

A device for measuring and diagnosing the galvanic skin response during common work with a computer (4) controlled by a surface-modified mouse (1), which is equipped with a control processor (11), comprising of four touch sensors made by contact electrodes (21) to (24) located on the surface of the mouse (1) at places of the correct hand grip of the mouse, i.e. two locations for the palm, one for the ring finger and one for the thumb, and interconnected with each other to form six measuring channels (K1) to (K6), wherein the mouse (1) is equipped with a signal processor (3) comprising of a measuring unit (31) which is divided into sections, the number of which corresponds to the number of measuring channels (K1) to (K6), where the measuring unit (31) is connected in series with a microprocessor unit (32) and a channel switch (33) such that each section of the microprocessor unit (32) is connected to one of the measuring channels (K1) to (K6) and the channel switch (33) is also connected back to the measuring unit (31) so that each of its sections is connected by a separate input, while the microprocessor unit 32 is further provided with an output (321) for connection to the control computer (4).

## Description

### Field of the Invention

The invention is within the field of measurement and evaluation of human physiological manifestations and relates to the construction of a device for measuring and diagnosing the galvanic skin response of a user while he/she operates a computer, and to the method of measuring and performing diagnostics by this device.

### Prior Art

The galvanic skin response is one of the basic psychophysiological signals, the scanning and evaluation of which is widely used in the field of human psychological analysis, also in specialized fields such as criminology, etc. Basic principles and theoretical bases are well described in published works, for example. M. Malmuvio, J., Plansey, R., Bioelectromagnetism: Principles and Applications of Bioelectric and Biomagnetic Fields, Oxford University Press, New York, 1995; or Boucsein, W., Electrodermal activity, Springer, 2012. Application of skin conductivity measurement for psychological and physiological analysis of people is described in Ischenko, A. N., Shevev, P. P., Biomedical Engineering, 23/3/1989, 113-117, Springer, New York; or Lustig, Z., et al., The New Educational Review, 21/2/2010, 97-110. The actual method is based on a proven assertion that mental, physical or emotional stimuli affect the activity of the human sweat glands in the skin, which are filled with sweat due to these stimuli. Sweat is a weak electrolyte and a good conductor, which leads to an increased conductivity of the skin. This phenomenon is described as the galvanic skin response (GSR) and consists of two parts, namely tonic and phasic. The tonic part is a low-frequency basic level of skin conductivity that can oscillate over days, its magnitude most often ranges from 2-20 µs and is highly individual. The tonic response of the skin increases with the expectation of various tasks, such as mental arithmetic, attention tasks, or discussing social problems, and also during the performance of activities accompanying them. The phasic part is on top of the tonic part, it has a higher frequency that increases together with the amplitude in moments of excitement. It reflects the level of stress and the degree of social empathy and other social emotions. These facts can be applied in the diagnosis and analysis of the conductivity of the skin on which a meter is attached in the form of a sensor or electrode. The data on the two basic parts of the skin conductivity is obtained by evaluation of the signals from the sensor and are further processed. For the low-frequency tonic conductivity, which is characterized by a long period of change and generally only slowly increases with the level of human excitement, it is necessary to calibrate the system for a particular person due to significant differences between individuals. The phasic part of the signal is usually evaluated based on the amplitude and latent period. A proportional tonic degree is often used to evaluate the amplitude of the phasic reaction in percent. The signals of both parts are not regular, their spectra are continuous and overlapping. However, the separation of the two signals is possible due to the fact that most of the energy of the tonic part is in the range of 0-0.05 Hz, while most of the energy of the phasic phase is in the range of 0.05-2 Hz.

The GSR measurement methods and systems used to monitor the psychological and psychophysiological states are known. US Patent No. 8882669 discloses a basic method of analyzing emotions and changes of mental states using GSR measurements by sensors placed on fingers. US Patent No. 6167299 discloses a classic GSR measurement system supplemented with a special signal processing to maximize the elimination of disturbances caused, in particular, by the movement of the person being diagnosed.

The problem of elimination and maximum filtering of interfering signals is one of the main problems of using the GSR method. It is not possible to use special gels used in ECG and EEG measuring, etc. as they reduce the transitional resistances between the skin and the electrodes, thus significantly affecting the measured conductivity values, literally masking the actual signal, which is not desirable. Insufficient contact of the electrodes with the skin must be solved mainly by means of signal processing, the introduction of reference electrodes and multi-channel measurement. UA 79668 discloses a solution for the elimination of interfering signals by means of measurements performed on the left and right hand simultaneously with subsequent comparison and analysis of both signals. US Patent No. 4144888559 discloses a method of multi-channel conductivity measurement and subsequent GSR analysis using electrode duplication to obtain a four-channel signal system which greatly reduces undesirable interference. The actual measurements are made by an alternating sinusoidal voltage of 200 Hz. However, this solution does not address another potential problem, which is the loss of skin contact with one or more electrodes. This problem is solved in CN 203815454, which discloses a GSR measuring apparatus which is applied to the wrist and contains an entire matrix of electrodes, wherein the measurement is carried out through multiple electrode combinations, i.e. as a multi-channel. In this case, however, the device uses a special capacitance non-contact sensor that requires specific conditions of use and cannot be generally applied in GSR measurement devices.

A computer mouse is a pointing device most commonly used as an input device for controlling computer programs, which also appears in a number of different specialized designs. E.g. US Patent No. 3277245 discloses an ergonomic mouse for reducing fatigue, pain and hand disability; another example is a specialized mouse for playing computer games. Using a computer mouse to place pressure sensors for measuring GSR during common computer work is known from a number of solutions that allow for the observation of the physiological state in connection with responses to various types of computer applications. US Patent No. 2006135857 discloses the use of a computer mouse as an input device for measuring various physiological variables including conductivity of the skin. US Patent No. 5741217 discloses the use of a computer mouse specifically for measuring GSR when working with computer applications. US Patent No. 6190314 discloses a system for monitoring physiological characteristics, including GSR, for evaluating emotions by means of sensors placed on the body of a computer mouse, including special software for evaluating emotional curves and characteristics that allows calibration and analysis of biophysical signals and variables. It is known that when using a computer mouse to measure the GSR, it is necessary to solve the problem of the uneven grip of the mouse and inadequate contact of the pressure sensors with the skin. This problem is partially solved in Patent GB 2378762, which describes the removal of the influence of unequal pressure on the sensor by using a correction signal which is obtained by continuous evaluation of the pressure on the respective sensor. However, this solution only uses two conductivity electrodes that allow only one-channel measurement. Another solution of the inadequate contact of the pressure sensors is given in the file CN 106909237, where the problem is solved by placing further pressure sensors on the mouse pad. The signal measured on the pad thus complements the insufficient signal from the measurements made on the computer mouse.

A goal of the claimed invention is to provide a novel device for measuring and diagnosing a skin galvanic response during computer work, and a method for measuring and performing diagnostics with this device; the device is based on the use of multi-channel measurements using four pressure sensors suitably placed on a computer mouse, which minimizes measurement disturbances due to insufficient contact of the measuring electrodes with the skin.

### Summary of the Invention

The stated goal is achieved by an invention which is a device for measuring and diagnosing a skin galvanic response of a person working with a computer that is controlled by a surface-modified mouse equipped with a control processor, characterized by four touch sensors on its surface, formed by contact electrodes which are placed on the locations of the correct hand grip of the mouse, .i.e. two locations for the palm, one for the middle finger and one for the thumb, and which are interconnected with each other, creating six measuring channels, wherein the mouse is equipped with a signal processor comprising of a measuring unit divided into sections in the amount that corresponds with the number of measuring channels; the measuring unit is connected in series with a microprocessor unit and channel switch, so that each section of the microprocessor unit is connected with one measuring channel and the channel switch is connected back to the measuring unit so that each of its section is connected with a single line-in and the microprocessor unit has an output for connecting it with a controlling computer.

In an advantageous variant, each section of the measuring unit comprises of in series connected R/U converter, amplifier, A/D converter, and galvanic separator, which comprises of an optocoupler, and the control and signal processors are connected with the controlling computer via a USB interface.

Another essence of the invention is a method of measuring and performing a diagnosis of a skin galvanic reaction of a person working with a computer operated by a mouse with its surface equipped with touch sensors, the contact electrodes, in which six channels are formed by the interconnection of the four contact electrodes located at the places of correct hand grip of the mouse, i.e. two locations for the palm, one for the ring finger and one for the thumb; the measuring current between the two selected contact electrodes is cyclically supplied by the signal processor, and the resulting signal in the form of a voltage proportional to the size of the respective GSR is amplified and digitized and after being galvanically separated from interference of other devices delivered to the software for evaluation and processing.

The new invention achieves a greater effect, as such type of device allows to obtain higher quality GSR results which are not affected by external influences, in particular, the effect of mishandling the mouse, because the six-channel measurement between the four contact points of the skin allows to process data with subsequent algorithmic evaluation, virtually eliminating measurement disturbances.

### Brief Description of the Drawings

Specific examples of implementation of the invention are depicted in the embodiments, wherein:
Fig. 1 shows a general view of the computer mouse equipped with sensors;
Fig. 2 shows the block diagram of the device;
Fig. 3 shows the internal scheme of the GSR signal processor;
Fig. 4 shows the organization of measuring for one channel;
Fig. 5 shows the diagram of the interconnection of the individual channels between the contact electrodes;
Fig. 6 shows the diagram of GSR measuring across the individual channels over time.

The exemplary embodiments that show the invention and the examples of specific implementations described hereafter should not be construed to be limitative in any manner and serve merely as illustration.

### Examples of implementation of the invention

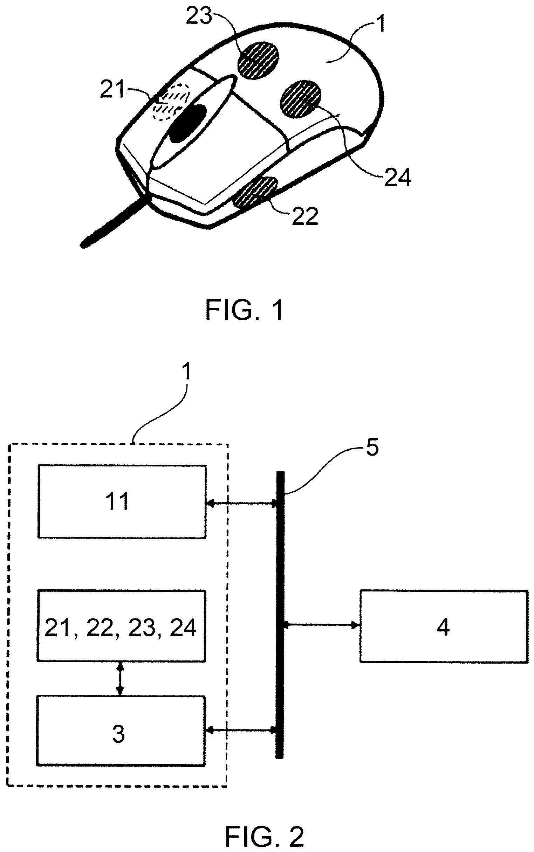

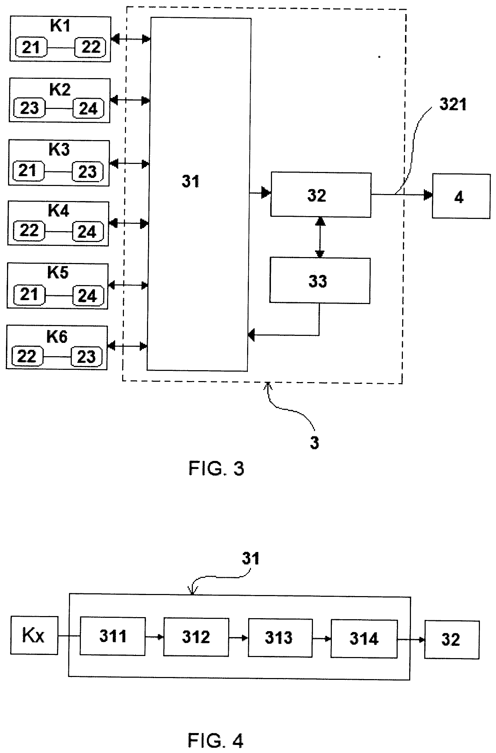

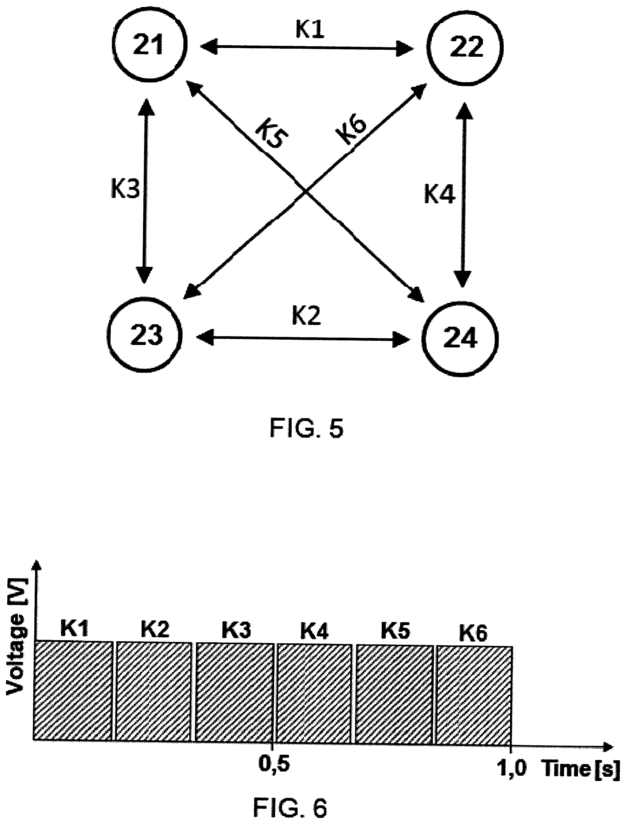

The device according to the invention comprises of a specially adapted computer mouse 1 which has four touch sensors on its surface made by contact electrodes 21, 22, 23, 24, at the locations of the assumed correct grip of the mouse 1, which are two locations for the palm, one for the ring finger and one for the thumb as can be seen in Fig.1. In addition to the standard control processor 11, the mouse 1 is equipped with another signal processor 3 which is connected with the contact electrodes 21, 22, 23, 24. The control processor 11 and the signal processor 3 are then connected to the host control computer 4, for example, via a serial bus 5 (Universal Serial Bus) interface as shown in Fig. 2. The contact electrodes 21, 22, 23, 24 are interconnected to one another forming six measuring channels K1, K2, K3, K4, K5, K6, as shown in Fig. 5. The signal processor 3 consists of a measuring unit 31 divided into sections corresponding to the number of measuring channels K1, K2, K3, K4, K5, K6. After the measuring unit 31, the microprocessor unit 32 and the channel switch 33 are connected in series, and to each microprocessor unit 32 section, there is one measuring channel K1 to K6 connected. The channel switch 33 is also connected in a feedback loop to the measuring unit 31 so that each of its sections is connected by a separate input, as can be seen from Fig. 3. The microprocessor unit 32 is further provided with an output 321 for connection to the control computer 4. Each section of the measuring unit 31 is in the basic implementation formed by a R/U converter 311, an amplifier 312, an A/D converter 313 and a galvanic separator 314 based on an optocoupler which are all connected in series as seen in Fig. 4.

In the measurement and evaluation of human physiological manifestations by means of the GSR analysis, the signal processor 3 carries out six mutually independent single-channel measurements using the channel switch 33 to bring a measuring current exactly during a predetermined time point, for example 0.15 to 0.20 s between the two selected contact electrodes 21 to 24 creating the measuring channels K1 to K6. This measuring current activates the R/U converter 311 of the respective section of the measuring unit 31, the R/U converter creates a voltage proportional to the size of the GSR signal. The voltage at the R/U converter 311 output is amplified by the amplifier 312 and then converted by the A/D converter 313 to a digital signal. Through the optocoupler-based galvanic separator 314, the digital signal from the measuring unit 31 is brought for evaluation and input processing into the microprocessor unit 32 from which the signal may be further transmitted to the control computer 4. This process is periodically repeated by switching the channel switch 33 between all six measuring channels K1 to K6, as shown in Fig. 6. This process provides a practically continuous flow of relevant data for subsequent GSR evaluation.

### Industrial applicability

The claimed invention is applicable for analyzing the emotional and psychophysiological characteristics of a person during common computer work and allows to remotely assess and indicate any possible medical or psychological complications. The invention can also be used to improve the feedback of electronic teaching programs, computer games and other interactive applications.

## Claims

1. A device for measuring and diagnosing the galvanic skin response of a person during common work with a computer (4) controlled by a surface-modified mouse (1), which is equipped with a control processor (11), **comprising of** four touch sensors formed by contact electrodes (21) to (24) located on the surface of the mouse (1) at places of the correct hand grip of the mouse, i.e. two locations for the palm, one for the ring finger and one for the thumb, and interconnected with each other to form six measuring channels (K1) to (K6), wherein the mouse (1) is equipped with a signal processor (3) comprising of a measuring unit (31) which is divided into sections, the number of which corresponds to the number of measuring channels (K1) to (K6), where the measuring unit (31) is connected in series with a microprocessor unit (32) and a channel switch (33) such that each section of the microprocessor unit (32) is connected to one of the measuring channels (K1) to (K6) and the channel switch (33) is also connected back to the measuring unit (31) so that each of its sections is connected by a separate input, while the microprocessor unit 32 is further provided with an output (321) for connection to the control computer (4).

2. Device introduced in Claim 1 **comprising of** several sections of the measuring unit (31) of which each section comprises of R/U converter (311), amplifier (312), A/D converter (313) and galvanic separator (314).

3. Device according to Claim 2 **comprising** of an optocoupler-based galvanic separator (314).

4. Device according to any and all of the Claims 1 to 3 **comprising of** a control processor (11) and a signal processor (3) which are connected with a host control computer (4) over a universal serial bus (USB) (5).

5. A method for measuring and diagnosing a galvanic skin response of a person working with a computer that is controlled by a mouse equipped with surface sensors formed by contact electrodes on a device according to claims 1 to 4, and **comprising of** up to six channels (K1) to (K6) formed by interconnecting the four contact electrodes (21) to (24) located on the surface of the mouse (1) at places of the correct hand grip of the mouse, i.e. two locations for the palm, one for the ring finger and one for the thumb, to which a signal processor (3) cyclically delivers a measuring current to a selected two contact electrodes (21) to (24) and the resulting signal in the form of a voltage proportional to the size of the respective GSR is digitized after the amplification and is then taken to the software evaluation and processing after galvanic isolation from other devices.
